# EUROPÄISCHE PATENTSCHRIFT

(11) **EP 1 385 816 B1**
(45) Veröffentlichungstag und Bekanntmachung des Hinweises auf die Patenterteilung: **15.04.2009**
(21) Anmeldenummer: 02745251.5
(22) Anmeldetag: 22.04.2002
(51) Int. Cl.: C07C 51/573, C07C 51/215, C07C 57/145

(54) **VERFAHREN ZUR REINIGUNG EINES ORGANISCHEN LÖSUNGSMITTELS ZUR ABSORPTION VON MALEINSÄUREANHYDRID**
METHOD FOR PURIFYING AN ORGANIC SOLVENT FOR THE PURPOSE OF ABSORPTION OF MALEIC ACID ANHYDRIDE
PROCEDE DE PURIFICATION D'UN SOLVANT ORGANIQUE DESTINE A L'ABSORPTION DE L'ANHYDRIDE DE L'ACIDE MALEIQUE

(30) Priorität: 23.04.2001 DE 10119737
(43) Veröffentlichungstag der Anmeldung: 04.02.2004
(73) Patentinhaber: BASF SE, 67056 Ludwigshafen (DE)
(72) Erfinder: RAHN, Ralf-Thomas, 68167 Mannheim (DE); WECK, Alexander, 67251 Freinsheim (DE); KAIBEL, Gerd, 68623 Lampertheim (DE)
(74) Vertreter: Pohl, Michael Friedrich
(86) Internationale Anmeldenummer: PCT/EP2002/004414
(87) Internationale Veröffentlichungsnummer: WO 2002/085834

(56) Entgegenhaltungen:
- EP-A- 0 459 543
- US-A- 4 118 403

## Beschreibung

Die Erfindung betrifft ein Verfahren zur Reinigung eines organischen Lösungsmittels zur Absorption von Maleinsäureanhydrid aus einem gasförmigen Gemisch.

Bei der technischen Herstellung von Maleinsäureanhydrid (MSA), die heute überwiegend durch Oxidation von n-Butan mit Luft oder Sauerstoff in der Gasphase erfolgt, wird MSA aus dem Oxidationsabgas durch Absorption vom restlichen Abgas getrennt. Die Absorption kann in Wasser oder, wie nach allgemeiner Auffassung noch günstiger, in einem organischen Lösungsmittel erfolgen. Geeignete Lösungsmittel sind beispielsweise in der WO 96/29323 beschrieben.

Nach erfolgter Absorption wird die MSA-enthaltende Lösung weiter verarbeitet. Zur Herstellung von Roh-MSA schließt sich eine ein- oder mehrstufige Strippung an, bei der Roh-MSA am Kolonnenkopf anfällt. Diese Strippung erfolgt entwederbei vermindertem Druck, vorzugsweise bei 50 bis 100 mbar oder unter Einsatz von Stickstoff, Wasserstoff oder einem Gemisch von Stickstoff und Wasserstoff als Strippgas. Am Sumpf der Kolonne fällt das weitgehend von MSA befreite Absorptionsmittel an und wird in die Absorption rückgeführt. Mit zunehmender Zahl der Rückführungen beobachtet man allerdings eine Aufpegelung von Maleinsäure und Fumarsäure. Desweiteren wird die Bildung weiterer saurer Bestandteile beobachtet, wie z. B. Monoalkylphthalate und Phthalsäureanhydrid. Außerdem wird die Bildung teerartiger Hochsieder festgestellt. In der WO 96/29323 wird die Entfernung der teerartigen Komponenten und Hochsieder durch Extraktion mit Wasser beschrieben. In den aufgeführten Versuchen wird ein Abreicherungsgrad von maximal 50 % erreicht, wobei insbesondere bei den sehr hohen Abreicherungsgraden die Bildung von Emulsionen beobachtet wurde, wodurch sich Probleme bei der Phasentrennung ergaben.

In der EP-A-897 905 ist die Extraktion von sauren Komponenten bei der Herstellung von MSA mittels wässriger alkalischer Lösungen beschrieben. Nach dieser Arbeitsweise wird ein Reaktionsmischungsgas, das Maleinsäureanhydrid enthält, in Kontakt mit einem organischen Lösungsmittel gebracht, mindestens ein Teil des Maleinsäureanhydrids vom organischen Lösungsmittel abgetrennt, ein Teil des organischen Lösungsmittels nach der Abtrennung des Maleinsäureanhydrids mit einer wässrigen Alkalilösung, beispielsweise Natronlauge oder Ammoniaklösung, gewaschen und das gewaschene organische Lösungsmittel und das verbliebene organische Lösungsmittel wieder in die Absorption des Maleinsäureanhydrids aus dem Reaktionsmischungsgas rückgeführt. Im Vergleich zur Extraktion mit reinem Wasser soll eine Extraktion mit Alkali zu besseren Ergebnissen führen. Über Probleme bei der Phasentrennung bei gleichzeitiger Anwesenheit von Hochsiedern wird in der Druckschrift nichts ausgeführt.

Nachteilig bei den in den obigen Druckschriften beschriebenen Verfahren ist der relativ niedrige Abreicherungsgrad, insbesondere bei teerartigen Polymeren. Ebenso nachteilig sind Schwierigkeiten bei der Phasentrennung, wenn der extrahierte Anteil der Hochsieder in die Nähe von 50 % kommt. Nachteilig ist auch der Umstand, dass die Wasserphase entsorgt werden muss. Eine Abgabe ins Abwasser ist möglicherweise problematisch, da das organische Material in der Wasserphase eine vorgegebene Abbaubarkeit aufweisen muss. Eine Verbrennung ist insbesondere dann nachteilig, wenn in der Wasserphase noch Alkalisalze enthalten sind.

Neben der Entsorgungsproblematik für die wässerige Phase führt der Wassergehalt der organischen Phase ebenfalls zu einem Problem. In der Regel führt ein höherer Wassergehalt im Lösungsmittel bei der MSA-Absorption zu erhöhter Bildung von Maleinsäure, so dass neben der Extraktionsstufe, die in der Regel aus einer Mixer-Settler-Kaskade besteht, noch Bedarf für eine Verdampfungsstufe zur Abtrennung von Restwasser auftreten kann.

In der US-A-4,118,403 ist ein Verfahren zur Reinigung eines organischen Lösungsmittels zur Absorption von Maleinsäureanhydrid aus einem gasförmigen Gemisch beschrieben, bei dem man das Maleinsäureanhydrid vom Lösungsmittel abtrennt, dem Lösungsmittelstrom einen Teilstrom entnimmt, diesen destilliert und in den Lösungsmittelkreislauf rückführt. Wie der Figur der Druckschrift zu entnehmen ist, wird der gesamte Lösungsmittelstrom nach der Abtrennung vom Maleinsäureanhydrid in der Strippkolonne 16 in einer Kühl/ Filtrationseinrichtung 41 herunter gekühlt und filtriert. Daran anschließend wird ein Teilstrom über die Destillationseinrichtung 42 ausgeschleust und zur Abtrennung von den Hochsiedern destilliert. Das Destillat wird dann in den Lösungsmittelkreis zurück geführt.

Nachteilig am bekannten Verfahren ist, dass sich Maleinsäure und Fumarsäure im Lösungsmittelkreislauf aufpegeln und sich somit der Säuregehalt erhöht. Durch die Anwesenheit von Säure wird die Umsetzung von Maleinsäureanhydrid mit Wasser zu Maleinsäure gefördert. Hierdurch nimmt die Ausbeute an MSA ab. Diese Befunde wurden in eigenen Laborversuchen festgestellt.

Der Erfindung liegt die Aufgabe zugrunde, die aus dem Stand der Technik bekannten Nachteile zu vermeiden und insbesondere eine Aufpegelung von sauren Komponenten im Lösungsmittelstrom zu vermeiden.

Diese Aufgabe wird gelöst mit einem Verfahren mit den Merkmalen des Oberbegriffs des Anspruchs 1, das dadurch gekennzeichnet ist, dass man eine Leichtsiederfraktion und eine Hochsiederfraktion von der im Wesentlichen aus gereinigtem Lösungsmittel bestehenden Fraktion abtrennt und die im wesentlichen aus gereinigtem Lösungsmittel bestehende Fraktion dem Lösungsmittelstrom wieder zuführt.

Erfindungsgemäß gelingt es nicht nur, die Hochsieder, sondern auch die sauren Verbindungen Maleinsäure, Fumarsäure, Phthalsäure etc. vor der Rückführung des Lösungsmittels in die Stufe der Absorption des gasförmigen MSA abzutrennen. Dies gelingt mit geringem apparativem Aufwand und ohne Einsatz von Fremdstoffen. Die im erfindungsgemäßen Verfahren abgetrennte Leichtsiederfraktion enthält als wesentliche Komponenten Maleinsäure, Fumarsäure, Phthalsäure sowie geringe Mengen an Maleinsäureanhydrid und eingesetztes Lösungsmittel. Die Hochsiederfraktion enthält als wesentliche Komponenten teerartige Produkte sowie geringe Mengen an eingesetztem Lösungsmittel. Die im Wesentlichen aus gereingtem Lösungsmittel bestehende Fraktion enthält das Lösungsmittel in einem Reinheitsgrad, der die unmittelbare Rückführung in den Lösungsmittelkreislauf erlaubt.

Das erfindungsgemäße Verfahren kann kontinuierlich, halbkontinuierlich oder diskontinuierlich durchgeführt werden.

Bei einer ersten, kontinuierlichen Ausführungsform des erfindungsgemäßen Verfahrens wird ein Teilstrom des verunreinigten Lösungsmittels einer Destillationskolonne zugeführt. Hierbei gehen über Kopf die leichtsiedenden sauren Komponenten Maleinsäureanhydrid, Maleinsäure, Fumarsäure und Phthalsäureanhydrid über, über den Sumpf werden die teerartigen Komponenten abgezogen und über den Seitenabzug erhält man das weitgehend Nebenprodukt-freie Lösungsmittel, beispielsweise Dibutylphthalat. Man destilliert zweckmäßig im Vakuum, vorzugsweise bei einem Druck von 0,1 bis 100 mbar, vorzugsweise bei einem Druck von 1 bis 20 mbar, besonders bevorzugt bei einem Druck von 2 bis 15 mbar. Bei Verwendung einer üblichen Kolonne wird das gereinigte Lösungsmittel, z. B. Dibutylphthalat, in der Regel im Abtriebsteil abgezogen. Daher wird der Seitenabzug gasförmig sein.

Für die Trennung werden im Allgemeinen etwa 1 bis 60, insbesondere etwa 10 bis 50 theoretische Trennstufen, vorzugsweise 15 bis 40, besonders bevorzugt 15 bis 30 theoretische Trennstufen, benötigt. Eine Definition der theoretischen Trennstufen findet sich z. B. in Klaus Sattler, Thermische Trennverfahren, S. 5, rechte Spalte, Absatz 2, ISBN 3-527-26727-1. Der Seitenabzug liegt zweckmäßig 1 bis 5 theoretische Trennstufen oberhalb des Sumpfes, vorzugsweise 1 bis 4 Trennstufen.

Nach einer Variante kann die Destillation in einer Trennwandkolonne erfolgen, wie in US-A-2,471,134, der EP-A-0 122 367 oder von G. Kaibel, Chem. Eng. Technol. Vol. 10, 1987, S. 92-98 beschrieben. In diesem Fall erfolgt die Abtrennung des Lösungsmittels, beispielsweise des Dibutylphthalats, in einem flüssigen Seitenabzug.

Als Verdampfer eignet sich ein verschmutzungsunempfindlicher und leicht zu reinigender Wärmetauscher, beispeilsweise ein Fallfilmverdampfer oder ein Dünnschichtverdampfer, ein Zwangsumlauf-Entspanungsverdampfer oder ein Rührkessel mit Rührvorrichtung, beispielsweise einem wandgängigen Ankerrührer.

Um am Kopfkondensator durch Aufkonzentrieren einen Feststoffanfall in Form von Maleinsäure bzw. Fumarsäure zu vermeiden, sollte der Kopfkondensator unempfindlich gegen Feststoffausfall ausgeführt werden. Hierzu bietet sich ein Quench-Kreislauf oder ein berieseltes Wärmetauscherbündel an. Auch die Kolonne sollte mit Feststoff-unempfindlichen Einbauten ausgestattet werden, beispielsweise mit Dual-Flow-Böden oder einer Blechpackung.

Somit ist die erste Ausführungsform des erfindungsgemäßen Verfahrens dadurch gekennzeichnet, dass man jeweils kontinuierlich die Leichtsiederfraktion über den Kopf, die Hochsiederfraktion über den Sumpf und das gereinigte Lösungsmittel gasförmig oder flüssig über den Seitenabzug einer Destillationskolonne abzieht.

Nach einer zweiten Ausführungsform des erfindungsgemäßen Verfahrens wird ebenfalls kontinuierlich gearbeitet. Anders als bei der ersten Ausführungsform erfolgt hier das Abtrennen der teerartigen Hochsieder in einem vorgelagerten Verdampfer. Anschließend wird das Destillat zur Abtrennung der leichtsiedenden sauren Bestandteile einer Destillation zugeführt. Im Unterschied zur ersten kontinuierlichen Ausführungsform wird hier das Lösungsmittel, beispielsweise Dibutylphthalat, am Sumpf der Kolonne abgezogen. Die Verdampfung im Vorverdampfer sowie die Destillation erfolgen zweckmäßig im Vakuum bei einem Druck von beispielsweise 0,1 bis 100 mbar, bevorzugt bei einem Druck von 1 bis 20 mbar, besonders bevorzugt bei einem Druck von 2 bis 15 mbar. Für die Trennung benötigt man im Allgemeinen etwa 1 bis 60, insbesondere etwa 10 bis 50 theoretische Trennstufen, bevorzugt 9 bis 37, besonders bevorzugt 12 bis 27 theoretische Trennstufen.

Als vorgelagerter Verdampfer eignet sich ein verschmutzungsunempfindlicher und leicht zu reinigender Wärmetauscher, beispielsweise ein Fallfilmverdampfer oder ein Dünnschichtverdampfer, ein Zwangsumlauf-Entspannungsverdampfer oder ein Rührkessel mit Rühreinrichtung, zweckmäßig einem wandgängigen Ankerrührer. Obgleich man nicht im selben Maße wie im Vorverdampfer mit Feststoffausfall rechnen muss, sollte dieses Phänomen auch beim Kolonnenverdampfer berücksichtigt werden. Um am Kopfkondensator durch das Aufkonzentrieren einen Feststoffausfall von Maleinsäure bzw. Fumarsäure zu vermeiden, sollte der Kondensator unempfindlich gegen Feststoffausfall ausgeführt sein. Hierzu bietet sich ein Quench-Kreislauf oder ein berieseltes Wärmetauscherbündel an.

Die soeben beschriebene zweite Ausführungsform des erfindungsgemäßen Verfahrens ist somit dadurch gekennzeichnet, dass man jeweils kontinuierlich die Leichtsiederfraktion und die im Wesentlichen aus gereinigtem Lösungsmittel bestehende Fraktion von der Hochsiederfraktion abdestilliert und aus dem Destillat über Kopf die Leichtsiederfraktion und am Sumpf die im Wesentlichen aus gereinigtem Lösungsmittel bestehende Fraktion abtrennt.

Eine dritte Ausführungsform arbeitet diskontinuierlich. Hierbei wird auf einen Rührkessel mit Rührvorrichtung, vorzugsweise einem wandgängigen Ankerrührer, eine Kolonne gesetzt. Das verunreinigte Lösungsmittel wird im Rührkessel vorgelegt. Man destilliert im Vakuum zwei Fraktionen ab, eine Leichtsiederfraktion bestehend aus Maleinsäureanhydrid und Maleinsäure, Fumarsäure, Phthalsäureanhydrid und Lösungsmittel, beispielsweise Dibutylphthalat. Als Hauptfraktion geht das Lösungsmittel, beispielsweise Dibutylphthtalat, über Kopf. Nach dem Einengen kann man aus dem Rührkessel ein Gemisch aus Hochsiedern und Lösungsmittel, beispielsweise Dibutylphthalat, abziehen. Die diskontinuierliche Fraktionierung kann beispielsweise durch Absinken des Drucks und/oder Erhöhung der Temperatur erfolgen. Um die Kolonnenbelastung gleichmäßig zu halten, kann man das Destillat an einem Punkt entnehmen, zwischenspeichern und wieder in die Kolonne zurück führen.

Die vierte Ausführungsform des erfindungsgemäßen Verfahrens arbeitet halbkontinuierlich. Im Gegensatz zur Variante 3 wird während des Destillierens noch Einsatzstoff in den Rührkessel hinein gefahren. Über Kopf gehen die genannten Leichtsieder ab, über den Seitenabzug wird das Lösungsmittel, beispielsweise Dibutylphthtalat, abgezogen. Während des Destillationsvorgangs wird kein Sumpfprodukt abgezogen und reichert sich während des Vorgangs an. Sobald ein maximaler Sumpfstand überschritten wird, fährt das Eingangsventil zu und der Rührkesselinhalt wird nach dem Brechen des Vakuums abgelassen. Grundsätzlich kann man aber auch kontinuierlich arbeiten, indem man laufend oder in Intervallen Sumpfprodukt abzieht.

Beim erfindungsgemäßen Verfahren wird vorzugsweise ein Lösungsmittel eingesetzt, das einen höheren Siedepunkt als die Leichtsieder Maleinsäure, Fumarsäure und Phthalsäureanhydrid aufweist. Vorzugsweise verwendet man ein Lösungsmittel, das einen um 5° Kelvin höheren Siedepunkt, vorzugsweise 10° Kelvin höheren Siedepunkt, am meisten bevorzugt 30° Kelvin höheren Siedepunkt aufweist als die Leichtsieder Maleinsäure und Fumarsäure und Phthalsäureanhydrid, so dass diese Komponenten als Leichtsieder abgetrennt werden können. Schließlich sollte der Siedepunkt des Lösungsmittels so niedrig liegen, dass es sich bei technisch realisierbarem Vakuum ohne Produktzersetzung noch von den teerartigen Polymeren abtrennen lässt.

Als Lösungsmittel kommen in Betracht Dialkylphthtalate, beispielsweise solche mit 2 bis 8 Kohlenstoffatomen in jeder Alkylkette. Beispielhaft seien genannt Dimethylphthalat, Diethylphthalat, Dipropylphthalat, Diisopropylphthalat, Dibutylphthalat, Diisobutylphthalat, Dimethyldihydrophthalat, Diethyldihydrophthalat, Dipropyldihydrophthalat, Diisopropyldihydrophthalat, Dibutyldihydrophthalat, Diisobutyldihydrophthalat, Dimethyltetrahydrophthalat, Diethyltetrahydrophthalat, Dipropyltetrahydrophthalat, Diisopropyltetrahydrophthalat, Dibutyltetrahydrophthalat und Diisobutyltetrahydrophthalat.

Man kann aber auch Monoalkylphthalat-Verbindungen einsetzen, vorzugsweise solche mit 2 bis 8 Kohlenstoffatomen in der Alkylkette. Beispiele sind Monomethylphthalat, Monoethylphthalat, Monopropylphthalat, Monoisopropylphthalat, Monobutylphthalat, Monoisobutylphthalat, Monomethyldihydrophthalat, Monoethyldihydrophthalat, Monopropyldihydrophthalat, Monoisopropyldihydrophthalat, Monobutyldihydrophthalat, Monoisobutyldihydrophthalat, Monomethyltetrahydrophthalat, Monoethyltetrahydrophthalat, Monopropyltetrahydrophthalat, Monoisopropyltetrahydrophthalat, Monobutyltetrahydrophthalat und Monoisobutyltetrahydrophthalat. Außerdem geeignet sind beispielsweise Dimethylbenzophenon, Dichlorphenyloxid, Hexahydrophthalate sowie monoalkylsubstituierte Bernsteinsäuren mit 12 bis 16 Kohlenstoffatomen. Eine repräsentative Auswahl von Lösungsmitteln sind in der WO 96/29323 beschrieben, auf die hier ausdrücklich Bezug genommen wird.

Für eine erfolgreiche Reinigung des Lösungsmittels reicht es in der Regel aus, wenn ein Teilstrom des umlaufenden Lösungsmittels ausgeschleust wird. Im Allgemeinen beträgt dieser Teilstrom etwa 0,2 bis 1 Gew.-%, bevorzugt 0,3 bis 0,7 Gew.-%, jeweils bezogen auf das Gesamtgewicht des Lösungsmittels.

Der hier im Zusammenhang mit der aus gereinigtem Lösungsmittel bestehenden Fraktion verwendete Begriff "im Wesentlichen" bedeutet, dass die Fraktion aus weitgehend gereinigtem Lösungsmittel besteht. Der Reinigungsgrad des gereinigten Lösungsmittels kann variieren. Er sollte so beschaffen sein, dass während der Durchführung des Absorptionskreislaufs in dem im Kreislauf befindlichen Lösungsmittel keine nennenswerte Aufpegelung saurer Bestandteile stattfindet, da sonst die Gefahr besteht, dass die Ausbeute an MSA abnimmt. Zweckmäßig liegt der Reinigungsgrad des im Teilstrom gereinigten Lösungsmittels bei mindestens 95 Gew.-% oder darüber, vorzugsweise bei mindestens 97 Gew.-%, besonders bevorzugt bei mindestens 99 Gew.-%.

Im Falle der Verwendung von Mono- oder Dialkylphthalaten beträgt der Reinigungsgrad vorzugsweise mindestens 97 Gew.-%, besonders bevorzugt mindestens 99 Gew.-%.

Die Erfindung wird an Hand der nachfolgenden Beispiele weiter erläutert.

### Beispiel 1

In eine Kolonne mit 20 theoretischen Trennstufen wurde auf der Trennstufe Nr. 10 (gerechnet vom Kolonnenkopf) ein Mengenstrom von 10,0 kg/h mit einer Zusammensetzung von 0,5 Gew-% MSA, 1,0 Gew.-% Maleinsäure, 1,0 Gew.-% Fumarsäure, 0,4 Gew.-% teerartigen Hochsiedern, 1,0 Gew.-% Phthalsäureanhydrid und 96,1 Gew.-% Dibutylphthalat gegeben. Bei einem Kopfdruck von 10 mbar wurde am Kopf ein Strom von 0,8 kg/h abgezogen. Etwa 4 kg/h Rücklauf wurden wieder auf den Kolonnenkopf zurück geführt. Das Kopfprodukt hatte eine Zusammensetzung von 6,3 Gew.-% MSA, 56 Gew.-% Dibutylphthalat und je 12,5 Gew.-% Maleinsäure und Fumarsäure, sowie Phthalsäureanhydrid (PSA). Am Sumpf wurde bei einer Temperatur von 228 °C 0,1 kg/h eines Gemischs bestehend aus 73 Gew.-% Dibutylphthalat und 27 Gew.-% Hochsiedern entnommen. Am Seitenabzug, der sich auf der (von oben gerechnet) siebzehnten theoretischen Trennstufe befand, fielen 9,1 kg/h Dibutylphthalat mit einer Reinheit von 99,8 % an. Die Wiedergewinnungsrate von Dibutylphthalat betrug 95 %.

### Beispiel 2

In einen Rührkessel mit aufgesetzter Kolonne mit 20 theoretischen Trennstufen wurde ein Gemisch von 100 kg mit einer Zusammensetzung von 0,5 Gew.-% MSA, 1,0 Gew.-% Maleinsäure, 1,0 Gew.-% Fumarsäure, 0,4 Gew.-% teerartigen Hochsiedern, 1,0 Gew.-% Phthalsäureanhydrid (PSA) und 96,1 Gew.-% Dibutylphthalat gegeben. Anschließend wurde die Apparatur auf 10 mbar evakuiert. Über einen Schwenktrichter wurde ein Teilstrom des Destillats wieder auf die Kolonne gegeben. Bei einem Rücklaufverhältnis von 8 wurden zunächst 10 kg Vorlauf abgenommen. Während des Destillierens stieg die Kopftemperatur von 103 °C auf 190 °C an. Das Destillat wurde in einem Behälter gesammelt und hatte folgende Zusammensetzung: MSA und Maleinsäure: 13 Gew.-%; Fumarsäure 19 Gew.-%; Phthalsäureanhydrid 10 Gew.-%; Dibutylphthalat 68 Gew.-%. Anschließend wurde auf ein Rücklaufverhältnis von 2 umgeschaltet und es wurden bei 190 °C 86 kg Dibutylphthalat mit einer Reinheit von 99,6 % entnommen. Im Rührkessel verblieben 4 kg Dibutylphthalat mit ca. 10 Gew.-% Hochsiedern. Die Rückgewinnungsrate von Dibutylphhalat betrug 86 %.

## Patentansprüche

1. Verfahren zur Reinigung eines organischen Lösungsmittels zur Absorption von Maleinsäureanhydrid aus einem gasförmigen Gemisch, wobei man das Maleinsäureanhydrid vom Lösungsmittel abtrennt, dem Lösungsmittelstrom einen Teilstrom entnimmt, diesen destilliert und in den Lösungsmittelkreislauf rückführt, **dadurch gekennzeichnet, dass** man eine Leichtsiederfraktion und eine Hochsiederfraktion von der im Wesentlichen aus gereinigtem Lösungsmittel bestehenden Fraktion abtrennt und die im Wesentlichen aus gereinigtem Lösungsmittel bestehende Fraktion dem Lösungsmittelstrom wieder zuführt.

2. Verfahren nach Anspruch 1, **dadurch gekennzeichnet, dass** man bei einem Druck von 0,1 bis 100 mbar, insbesondere 1 bis 20 mbar destilliert.

3. Verfahren nach Anspruch 1 oder 2, **dadurch gekennzeichnet, dass** man jeweils kontinuierlich die Leichtsiederfraktion über Kopf, die Hochsiederfraktion über den Sumpf und das gereinigte Lösungsmittel gasförmig oder flüssig über den Seitenabzug einer Destillationskolonne abzieht.

4. Verfahren nach Anspruch 1 oder 2, **dadurch gekennzeichnet, dass** man jeweils kontinuierlich die Leichtsiederfraktion und die im Wesentlichen aus gereinigtem Lösungsmittel bestehende Fraktion von der Hochsiederfraktion abdestilliert, und aus dem Destillat über Kopf die Leichtsiederfraktion und am Sumpf die im wesentlichen aus gereinigtem Lösungsmittel bestehende Fraktion abtrennt.

5. Verfahren nach Anspruch 1 oder 2, **dadurch gekennzeichnet, dass** man diskontinuierlich oder halbkontinuierlich arbeitet.

6. Verfahren nach einem der Ansprüche 1 bis 5, **dadurch gekennzeichnet, dass** man ein organisches Lösungsmittel mit einem Siedepunkt verwendet, der bei Normaldruck mindestens 30° Kelvin über dem Siedepunkt der Leichtsiederfraktion liegt.

7. Verfahren nach einem der Ansprüche 1 bis 4 und 6, **dadurch gekennzeichnet, dass** man die Destillation unter Verwendung einer Trennwandkolonne durchführt.

## Claims

1. A process for purifying an organic solvent for the absorption of maleic anhydride from a gaseous mixture, where the maleic anhydride is separated from the solvent, a substream is taken from the solvent stream and this substream is distilled and returned to the solvent circuit, wherein a low-boiling fraction and a high-boiling fraction are separated off from the fraction consisting essentially of purified solvent and the fraction consisting essentially of purified solvent is fed back into the solvent stream.

2. The process according to claim 1, wherein the distillation is carried out at a pressure of from 0.1 to 100 mbar, in particular from I to 20 mbar.

3. The process according to claim 1 or 2, wherein the low-boiling fraction is taken off continuously at the top of a distillation column, the high-boiling fraction is taken off continuously from the bottom of the column and the purified solvent is taken off continuously in gaseous or liquid form via the side offtake of the column.

4. The process according to claim 1 or 2, wherein the low-boiling fraction and the fraction consisting essentially of purified solvent are distilled off continuously from the high-boiling fraction, and the low-boiling fraction is separated off from the distillate via the top and the fraction consisting essentially of purified solvent is separated off at the bottom.

5. The process according to claim 1 or 2 which is carried out batchwise or semicontinuously.

6. The process according to any of claims 1 to 5, wherein the organic solvent used has a boiling point which, at atmospheric pressure, is at least 30° Kelvin above the boiling point of the low-boiling fraction.

7. The process according to any of claims 1 to 4 and 6, wherein the distillation is carried out using a dividing wall column.

## Revendications

1. Procédé pour la purification d'un solvant organique en vue de l'absorption d'anhydride de l'acide maléique d'un mélange gazeux, où on sépare l'anhydride de l'acide maléique du solvant, on prélève un flux partiel du flux de solvant, on le distille et on le recycle dans le circuit du solvant, **caractérisé en ce qu'**on sépare une fraction à bas point d'ébullition et une fraction à point d'ébullition élevé de la fraction essentiellement constituée de solvant purifié et on réalimente la fraction essentiellement constituée de solvant purifié dans le flux de solvant.

2. Procédé selon la revendication 1, **caractérisé en ce qu'**on distille à une pression de 0,1 à 100 mbars, en particulier de 1 à 20 mbars.

3. Procédé selon la revendication 1 ou 2, **caractérisé en ce qu'**on soutire à chaque fois en continu la fraction à bas point d'ébullition via la tête, la fraction à point d'ébullition élevé via le fond et le solvant purifié sous forme gazeuse ou liquide via l'évacuation latérale d'une colonne de distillation.

4. Procédé selon la revendication 1 ou 2, **caractérisé en ce qu'**on élimine par distillation à chaque fois en continu la fraction à bas point d'ébullition et la fraction essentiellement constituée par le solvant purifié de la fraction à point d'ébullition élevé et on sépare du distillat, via la tête, la fraction à bas point d'ébullition et dans le fond la fraction essentiellement constituée de solvant purifié.

5. Procédé selon la revendication 1 ou 2, **caractérisé en ce qu'**on travaille de manière discontinue ou semi-continue.

6. Procédé selon l'une quelconque des revendications 1 à 5, **caractérisé en ce qu'**on utilise un solvant organique présentant un point d'ébullition qui est situé, à pression normale, au moins 30° Kelvin au-dessus du point d'ébullition de la fraction à bas point d'ébullition.

7. Procédé selon l'une quelconque des revendications 1 à 4 et 6, **caractérisé en ce que** qu'on réalise la distillation avec utilisation d'une colonne à paroi de partage.
